# EUROPÄISCHE PATENTSCHRIFT

(11) **EP 0 413 215 B1**
(45) Veröffentlichungstag und Bekanntmachung des Hinweises auf die Patenterteilung: **20.04.1994**
(21) Anmeldenummer: 90114910.4
(22) Anmeldetag: 03.08.1990
(51) Int. Cl.: C07C 68/00, C07C 69/96

(54) **Verfahren zur Herstellung von Dialkylcarbonaten**
Process for the preparation of dialkyl carbonates
Procédé de préparation de carbonates de dialkyle

(30) Priorität: 12.08.1989 DE 3926709
(43) Veröffentlichungstag der Anmeldung: 20.02.1991
(73) Patentinhaber: BASF Aktiengesellschaft, 67063 Ludwigshafen (DE)
(72) Erfinder: Joerg, Klaus, Dr., D-6703 Limburgerhof (DE); Mueller, Franz-Josef, Dr., D-6706 Wachenheim (DE); Harder, Wolfgang, Dr., D-6940 Weinheim (DE); Kummer, Rudolf, Dr., D-6710 Frankenthal (DE)

(56) Entgegenhaltungen:
- EP-A- 0 134 668
- US-A- 4 360 477

## Beschreibung

Die vorliegende Erfindung betrifft ein Verfahren zur kontinuierlichen Herstellung von Dialkylcarbonaten der allgemeinen Formel I 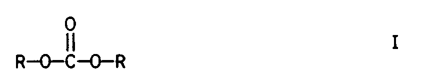
worin R eine C₁- bis C₄-Alkylgruppe ist, durch Umsetzung von C₁- bis C₄-Alkanolen mit Kohlenmonoxid und Sauerstoff in Gegenwart eines im Reaktionsmedium gelösten oder suspendierten, kupferhaltigen Katalysators, bei erhöhter Temperatur und bei erhöhtem Druck.

Dialkylcarbonate werden unter anderem zur Herstellung spezieller Monomere sowie zur Carbonylierung und Alkylierung hochveredelter organischer Zwischenprodukte verwendet. Beispielsweise dient Dimethylcarbonat als Ausgangsmaterial für Polycarbonate, als Wirkstoffzwischenprodukt und als Lösungsmittel für Cellulosederivate.

Zur Herstellung von Dialkylcarbonaten durch die katalytische Umsetzung der Ausgangsprodukte Alkanol, Kohlenmonoxid und Sauerstoff wurden in den vergangenen Jahren eine Reihe von Verfahren entwickelt.

Laut JP-45/11 129 (1970) werden zweiwertige Kupfersalze, insbesondere Kupfer(II)chlorid, als Katalysator verwendet. Die Ausbeute ist bei diesem Verfahren sehr gering und ein großer Teil des Methanols wird in der stark sauren Reaktionslösung - das Kupfersalz bildet mit dem Nasser stark saure Kationsäuren - in unerwünschte Nebenprodukte wie Methylchlorid und Dimethylether umgewandelt. Dieses Verfahren wird daher industriell nicht ausgeübt.

In DE-A 21 10 194 werden Kupferkatalysatoren verwendet, in denen das Kupfer mittels organischer Komplexbildner komplexiert wurde. Die Kupferionen können aufgrund der bei der Umsetzung ablaufenden Redoxreaktion nebeneinander in der Wertigkeitsstufe 1 und 2 vorliegen. Dieses Verfahren liefert im Laborversuch gute Ausbeuten, hat aber den Nachteil, daß diese Komplexkatalysatoren sehr teuer und gegenüber der Einwirkung von Wasser und Kohlendioxid empfindlich sind, wodurch es zu ihrer Zersetzung und zur Ausfällung von Kupfercarbonat kommt.

Weiterhin bereitet die Aufarbeitung der Reaktionsprodukte besondere Schwierigkeiten, weshalb dieses Verfahren gleichfalls nicht für eine Produktion im industriellen Maßstab geeignet ist.

Aus diesem Grunde werden in DE-A-27 43 690 an Stelle von Kupferkomplexverbindungen einfache Salze des einwertigen Kupfers als Katalysatoren benutzt. Zwar liefert auch dieses Verfahren gute Ausbeuten an Dialkylcarbonaten, wiederum bereitet jedoch die Aufarbeitung des Reaktionsproduktes im industriellen Maßstab große Probleme.

Die Abtrennung des Katalysators vom Reaktionsprodukt wird nach der Lehre des oben genannten Patentes durch Abfiltrieren oder Zentrifugieren im Falle des suspendierten Katalysators oder durch Rektifizieren und Kristallisieren bei gelöstem Katalysator erreicht. Da die katalysatorhaltigen Reaktionslösungen hochkorrosiv sind, erfordert die Aufarbeitung von Reaktionsprodukt und Katalysator einen hohen apparativen Aufwand: So müssen sämtliche Teile der Anlage - wie Kessel, Rohrleitungen, Destillations-, Kristallisations- und Filtrationsapparate - welche mit katalysatorhaltigen Lösungen in Kontakt kommen, mit korrosionsbeständigen Materialien wie Industriekeramik, Email, Teflon oder Tantal ausgekleidet werden und selbst dann sind Korrosionsprobleme, welche bei Pumpen und Ventilen auftreten, noch zu lösen. Dadurch wird das Verfahren unwirtschaftlich.

Die gleichen Aufarbeitungsprobleme machen auch das Verfahren der DE-A-30 45 767, welche sich von der vorgenannten Patentschrift nur durch die Verwendung von Synthesegas unterscheidet, wirtschaftlich unattraktiv. Die durch den Kupferkatalysator verursachten Korrosionsprobleme lassen auch die Übertragung der Verfahren von EP-A 217 651 (Kupfer(methoxy)chlorid-Katalysator unter Mitverwendung stickstoffhaltiger Cosolventien), US-A 4 638 076 (Phosphoramide als Cosolventien für den Kupferkatalysator), US-A 4 636 576 (cyclische Amide als Cosolventien), US-A 4 604 242 (Kupferpentandionat-Komplexe als Katalysatoren), US-A 4 370 275 (Kupferkomplexe mit Stickstoffbasen) und DE-A-23 34 736 (Kupferkomplexe mit organischen Phosphorverbindungen) in den industriellen Maßstab unwirtschaftlich werden.

Eine verfahrenstechnische Alternative zur Abtrennung des Katalysators in einem Katalysatorkreislauf wird in EP-A 134 668 gelehrt. Dabei wird im Reaktor das herzustellende Dialkylcarbonat zusammen mit dem Katalysator vorgelegt und in diese Suspension ein Gasstrom aus Kohlenmonoxid, Sauerstoff und Inertgas sowie das umzusetzende Alkanol kontinuierlich eingeleitet. Das bei der Umsetzung anfallende Wasser wird mit Hilfe des Dialkylcarbonats kontinuierlich azeotrop abdestilliert und das Wasser aus diesem Azeotrop abgetrennt. Da zur Bildung des Dialkylcarbonat-Wasser-Azeotrops mehr Dialkylcarbonat benötigt wird als sich bei der Umsetzung bildet, muß ein Teil des azeotrop abdestillierten Dialkylcarbonats wieder in den Reaktor zurückgeführt werden. Zur möglichst effektiven Gestaltung der Wasserabtrennung, d.h. also zur Vermeidung der energieaufwendigen Im-Kreis-Führung großer Mengen Dialkylcarbonats ist es erforderlich, daß die Alkanolkonzentration in der Reaktionsmischung möglichst niedrig, vorzugsweise unterhalb 5 Gew.-%, liegt. Die niedere Konzentration des Reaktionspartners Alkanol im Reaktionsmedium hat eine starke Verringerung der Raum-Zeit-Ausbeute zur Folge. So wird nur eine bescheidene Raum-Zeit-Ausbeute an Dimethylcarbonat von 15 g/(l·h) erzielt. Da US-A 4 360 477 praktisch nach dem gleichen Aufarbeitungsverfahren arbeitet, gelten hinsichtlich der Wirtschaftlichkeit dieses Verfahrens die gleichen Aussagen.

Es bestand somit die Aufgabe, ein Verfahren zur Herstellung von Dialkylcarbonat aus Alkanol, Kohlenmonoxid und Sauerstoff zu finden, das die wirtschaftliche Herstellung von Dialkylcarbonat erlaubt, das insbesondere hohe Raum-Zeit-Ausbeuten ermöglicht und sich ohne eine Kreislaufführung des korrosiven Katalysators, kontinuierlich im großtechnischen Maßstab durchführen läßt.

Dementsprechend wurde ein Verfahren zur kontinuierlichen Herstellung von Dialkylcarbonaten der allgemeinen Formel I 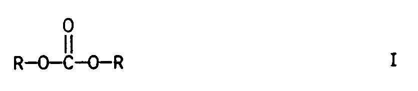
worin R eine C₁- bis C₄-Alkylgruppe ist, durch Umsetzung von C₁- bis C₄-Alkanolen mit Kohlenmonoxid und Sauerstoff in Gegenwart eines im Reaktionsmedium gelösten oder suspendierten, kupferhaltigen Katalysators, bei erhöhter Temperatur und bei erhöhtem Druck gefunden, das dadurch gekennzeichnet ist, daß man
a) einen Kohlenmonoxid und Sauerstoff enthaltenden Gasstrom in einer Menge von 1 bis 100 Nl/h pro g im Reaktor als Kupferkatalysator vorhandenen Kupfers durch das im Reaktor befindliche Alkanol-Katalysator-Gemisch leitet, wobei ein Teil dieses Gasstromes mit dem Alkanol zu Dialkylcarbonat und Wasser umgesetzt wird und mit Hilfe des restlichen, nicht umgesetzten Kohlenmonoxid/Sauerstoff-Gasstroms das gebildete Dialkylcarbonat und Wasser laufend zusammen mit dem Alkanol, gasförmig aus der Reaktionsmischung austrägt, anschließend
b) das erhaltene, gasförmige Gemisch in einem Abscheider in eine gasförmige und eine flüssige Phase zerlegt, die gasförmige Phase gewünschtenfalls wieder in den Reaktor zurückführt und
c) die im wesentlichen aus Dialkylcarbonat, Wasser und Alkanol bestehende flüssige Phase in ihre Bestandteile trennt, das Dialkylcarbonat isoliert, das Alkanol gewünschtenfalls wieder in den Reaktor zurückführt und
d) die Flüssigkeitsmenge im Reaktor durch Zuleitung frischen oder zurückgeführten Alkanols, nach Maßgabe des Alkanolverbrauches und -austrages ergänzt.

Die Dialkylcarbonate (I) bilden sich durch Umsetzung der Alkanole ROH mit Kohlenmonoxid und Sauerstoff, vorzugsweise in Gegenwart eines Kupferkatalysators formal gemäß Gleichung (1) (vgl. dazu auch: Saegusa et al; J. Org. Chem., 35 2976 (1970)) 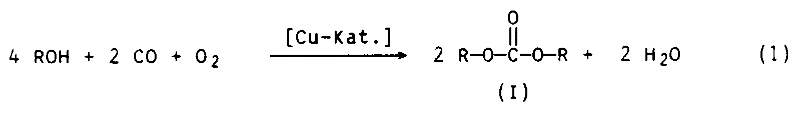
Im erfindungsgemäßen Verfahren wird das umzusetzende Alkanol im Reaktor zusammen mit dem Katalysator vorgelegt und die Reaktionsgase Sauerstoff und Kohlenmonoxid, gewünschtenfalls zusammen mit Inertgas, durch diese Reaktionsmischung geleitet. Das bei der Umsetzung gebildete Dialkylcarbonat und Wasser werden vom Gasstrom aus nicht abreagiertem Kohlenmonoxid und Sauerstoff aus der Reaktionslösung ausgetragen. Das flüssige Reaktionsmedium besteht im wesentlichen aus dem umzusetzenden Alkanol. Im allgemeinen beträgt das bezüglich des Alkanolgehaltes auf 1 normierte Molverhältnis Alkanol/Dialkylcarbonat/Katalysator (berechnet als Cu in der Reaktionsmischung) im Reaktionsmedium und während der Umsetzung 1/(0,03-1)/(0,01-0,33), vorteilhaft 1/(0,05-0,5)/(0,01-0,1) und besonders bevorzugt 1/(0,1-0,2)/(0,01-0,05).

Bei niedrigeren Katalysatorgehalten der Reaktionsmischung läßt sich die Umsetzung ebenfalls durchführen, wenn auch mit geringerer Raum-Zeit-Ausbeute, während sich bei höheren Katalysatorkonzentrationen die Wirtschaftlichkeit trotz eines höheren Aufwands an Katalysator nicht mehr weiter verbessert.

Hinsichtlich des Dialkylcarbonatgehaltes des Reaktionsgemisches wird zweckmäßigerweise danach getrachtet, das Dialkylcarbonat, sobald es gebildet wird, zusammen mit dem Reaktionswasser aus dem Reaktionsgemisch auszutragen, d.h. es wird im allgemeinen eine niedrige stationäre Dialkylcarbonatkonzentration im Reaktor angestrebt.

Bei höheren stationären Dialkylcarbonatkonzentrationen läßt sich die Umsetzung zwar auch durchführen, allerdings nur mit einem höheren Aufwand bei der Produktabtrennung und unter Inkaufnahme einer verringerten Raum-Zeit-Ausbeute.

Eine derart niedrige stationäre Dialkylcarbonatkonzentration in der Reaktionsmischung, einhergehend mit einer hohen Raum-Zeit-Ausbeute, wird erfindungsgemäß dadurch erzielt, daß man die Kohlenmonoxid- und Sauerstoffenthaltenden Reaktionsgase in einer Menge von Zweckmäßigerweise 1 bis 100, vorteilhaft 10 bis 50 und insbesondere 20 bis 30 Nl pro Stunde und pro Gramm als Kupferkatalysator im Reaktor vorhandenen Kupfers durch das Reaktionsgemisch leitet. Unter den Reaktionsbedingungen bildet sich nahezu ausschließlich aus einem Teil der Reaktionsgase und dem Alkanol Dialkylcarbonat und Wasser, während der restliche Teil der Reaktionsgase dazu dient, das gebildete Dialkylcarbonat zusammen mit dem Reaktionswasser und den entsprechend des thermodynamischen Dampf-Flüssig-Gleichgewichtes erforderlichen Mengen an Alkanol aus der Reaktionsmischung zu strippen.

Diese Vorgehensweise vereinfacht nicht nur die Isolierung des Verfahrensproduktes Dialkylcarbonat, sie wirkt sich auch günstig auf die Produktivität der Umsetzung durch eine vorteilhafte Verschiebung des Reaktionsgleichgewichtes aus. Sie hat außerdem eine niedrige stationäre Konzentration des Reaktionswassers in der Reaktionsmischung zur Folge. Diese beträgt im allgemeinen weniger als 5, vorteilhaft weniger als 1 Gew.-%, bezogen auf die Reaktionsmischung. Infolgedessen werden Nebenreaktionen wie die Bildung von Kohlendioxid und die sukzessive Desaktivierung des Kupferkatalysators unter Bildung von Kupfercarbonaten unterdrückt.

Es versteht sich von selbst, daß die Zufuhr der Reaktionsgase, welche im übrigen auch Inertgas zugemischt enthalten können, auch so gesteuert werden kann, daß die Konzentration des Alkohols im Reaktionsaustrag in sehr weiten Grenzen variiert werden kann, wobei ein hoher Alkoholgehalt im Austrag einen hohen Aufwand für die Energiekosten der Abtrennung haben kann.

Die Umsetzung der Reaktionsgase mit dem Alkanol kann bei Atmosphärendruck oder unter erhöhtem Druck vorgenommen werden. Im allgemeinen arbeitet man bei Drucken von 1 bis 50, bevorzugt von 15 bis 30 bar und bei Temperaturen von 80 bis 200°C, bevorzugt von 90 bis 130°C. Der Druck wird zweckmäßigerweise durch Aufpressen des Reaktionsgases eingestellt.

Das Kohlenmonoxid/Sauerstoff-Molverhältnis im Reaktionsgas kann in weiten Grenzen variiert werden, doch wird zweckmäßigerweise ein Kohlenmonoxid/ Sauerstoff-Molverhältnis von 1/0,01 bis 1/0,5 angewandt. Bei diesen Molverhältnissen ist zum einen der Sauerstoff-Partialdruck genügend groß, um wirtschaftliche Raum-Zeit-Ausbeuten zu erzielen, und zum anderen können sich aufgrund eines niedrigen Sauerstoffgehaltes keine explosionsfähigen Kohlenmonoxid/Sauerstoff-Gasgemische bilden.

Das Reaktionsgas - Kohlenmonoxid und Sauerstoff - kann auch noch Zusätze an Inertgas zugemischt enthalten. Dies ist insbesondere dann zweckmäßig, wenn mit ansonsten explosionsfähigen Kohlenmonoxid-Sauerstoff-Gasgemischen gearbeitet werden soll. Durch den Zusatz geeigneter Mengen an Inertgas wird eine Explosion dieser Mischungen verhindert.

Als Katalysatoren werden im erfindungsgemäßen Verfahren Kupferkatalysatoren auf der Basis von Kupfer(I)- und/oder Kupfer(II)-salzen verwendet. Da es sich bei der Umsetzung gemäß Gleichung (1) um eine Redoxreaktion handelt, liegen beide Kupferionenspezies nebeneinander in der Reaktionsmischung vor. Als Kupfersalz-Katalysatoren werden vorzugsweise einfache oder gemischte Kupfersalze wie Kupfer(I)- und/oder Kupfer(II)-halogenide, Kupfer(I oder II)-sulfat, Kupfer(II)(C₁-bis C₄-)(alkoxy)halogenide wie Kupfer(methoxy)chlorid, Kupfer(ethoxy)chlorid, Kupfer(butoxy)chlorid oder Kupfer(methoxy)bromid verwendet. Bei Verwendung von Kupfer(alkoxy)halogeniden als Katalysator entspricht zweckmäßigerweise deren Alkoxykomponente dem umzusetzenden Alkanol, da andernfalls gemischte Dialkylcarbonate gebildet werden, was im allgemeinen nicht erwünscht ist. Besonders bevorzugt werden im erfindungsgemäßen Verfahren Kupfer(I)halogenide, insbesondere Kupfer(I)chlorid und/oder die dem umzusetzenden Alkanol entsprechenden Kupfer(alkoxy)halogenide benutzt. Der Kupferkatalysator wird im allgemeinen in Mengen von 0,1 bis 10, vorteilhaft 0,3 bis 3, insbesondere von 0,3 bis 1,5 mol/l Alkanol eingesetzt. Da die katalytisch aktiven Kupfersalze in den Alkanolen nur mäßig löslich sind, führen die hier erwähnten Katalysatorkonzentrationen häufig zur Bildung von Katalysatoraufschlämmungen.

Beispielsweise können, unter den angegebenen Reaktionsbedingungen bei einer Einspeisung von 50 Nl CO/O₂-Gasgemisch, z.B. bei der Herstellung von Dimethylcarbonat, Raum-Zeit-Ausbeuten von 20 bis 75, vorteilhaft 40 bis 60 g/h und l Reaktionsmischung aus dieser ausgetragen und isoliert werden. Die Selektivität bezogen auf Methanol beträgt dabei 98 bis 100 %.

Das erfindungsgemäße Verfahren ist zur Herstellung von C₁- bis C₄-Dialkylcarbonaten wie Dimethylcarbonat, Diethylcarbonat, Di-n-propylcarbonat, Diisopropylcarbonat und Dibutylcarbonaten geeignet. Besonders vorteilhaft dient es zur Herstellung von Diethylcarbonat und insbesondere Dimethylcarbonat.

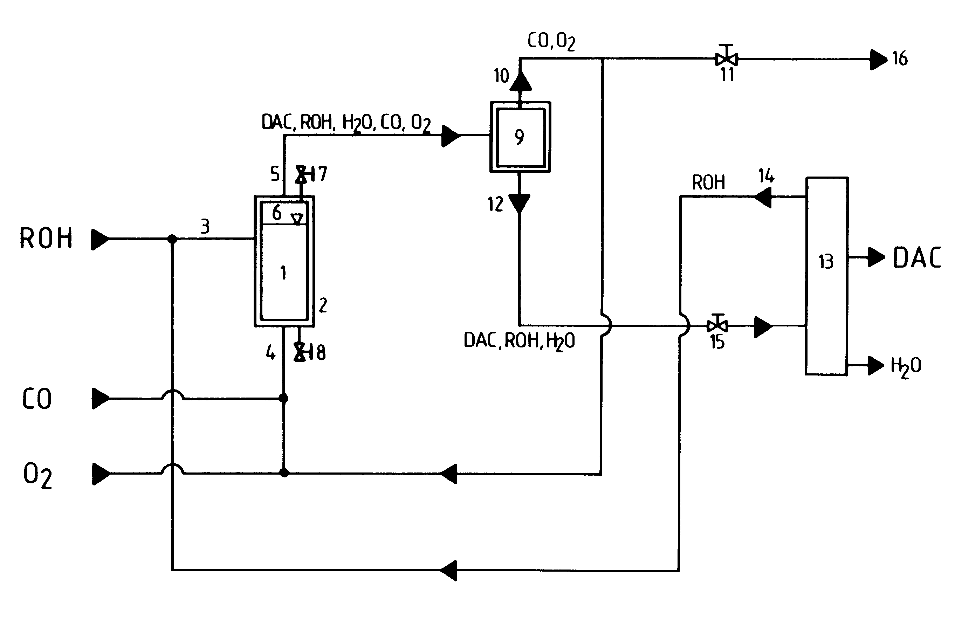

Anhand der Figur soll im folgenden das erfindungsgemäße Verfahren skizziert werden:
Die Umsetzung erfolgt im Reaktor 1, der beispielsweise mittels eines Heizmantels 2 von außen auf die Reaktionstemperatur beheizt wird und mit Zuleitungen 3 (für das Alkanol) und 4 (für das CO/O₂-enthaltende Reaktionsgas) sowie der Ableitung 5 für den gasförmigen Reaktionsaustrag versehen ist. Der Flüssigkeitsspiegel 6 im Reaktor kann so eingestellt werden, daß er sich ober- oder unterhalb der Alkanoleinleitung befindet; vorzugsweise wird das Alkanol (ROH) unterhalb des Flüssigkeitsspiegels eingeleitet. Der Katalysator kann über den Einfüllstutzen 7 in den Reaktor eingebracht und das katalysatorhaltige Reaktionsmedium gewünschtenfalls über den Ablaßstutzen 8 abgelassen werden.

Zur Umsetzung wird in den mit katalysatorhaltigem Alkanol beschickten und auf Reaktionstemperatur beheizten Reaktor über die am Boden des Reaktors angebrachte Einleitung 4 das CO/O₂-enthaltende Reaktionsgas eingeleitet. Zur besseren Dispergierung der Gase im flüssigen Reaktionsmedium kann dies vorteilhaft mit Hilfe von üblichen Dispergiervorrichtungen wie Prallblechen oder Düsen usw. erfolgen (vgl. DE-C 19 06 448; Nagel et al: Chem.-Ing. Techn., 42, 474 (1970); Zehner: Chem.-Ing.-Techn. 47, 209 (1975)). Der Reaktor kann wie ein Blasenreaktor betrieben werden. Gewünschtenfalls kann er zum besseren Inkontaktbringen von Reaktionsgas, Reaktionsmedium und Katalysator noch zusätzlich mit Rühreinrichtungen, Umpumpvorrichtungen oder dergleichen ausgestattet sein.

Das bei der Umsetzung gebildete Dialkylcarbonat (DAC) wird mit Hilfe des Kohlenmonoxid und Sauerstoff enthaltenden Reaktionsabgases zusammen mit dem Reaktionswasser und zusammen mit der zur Bildung des azeotropen Gemisches erforderlichen Menge an Alkanol über die Ableitung 5 dem Abscheider 9 zugeführt. In diesem Abscheider 9, bei dem es sich um einen druckstabilen Kühler handeln kann, wird der gasförmige Reaktionsaustrag unter Druck, zweckmäßigerweise unter dem Reaktionsdruck, in seine bei niedrigerer Temperatur flüssigen und gasförmigen Bestandteile aufgetrennt.

Der Druck im Abscheider 9 beträgt im allgemeinen 5 bis 125 % des Drucks im Reaktionsgefäß 1, bevorzugt wird die Abscheidung unter dem gleichen Druck wie die Umsetzung vorgenommen. Die Abscheidung könnte beispielsweise auch unter Atmosphärendruck vorgenommen werden; dies ist allerdings nicht wirtschaftlich. Die Abscheidung wird zweckmäßigerweise bei einer Temperatur von 40 bis -20°C, vorzugsweise bei 25 bis -15°C, vorteilhaft bei 10 bis -10°C durchgeführt.

Die gasförmigen Bestandteile werden über die Ableitung 10 am Kopf des Abscheiders abgezogen und gewünschtenfalls über den Einlaß 4 in die Umsetzung zurückgeführt. In diesem Falle wird zweckmäßigerweise der Kohlenmonoxid- und der Sauerstoffgehalt im zurückzuführenden Restgas nach Maßgabe des Verbrauchs durch Zudosieren frischen Kohlenmonoxids bzw. Sauerstoffs ergänzt. Der Druck kann bei dieser Kreisgasführung über das Druckregelventil 11 reguliert werden. Falls eine Rückführung des Restgases nicht gewünscht wird, kann es über den Auslaß 16 aus der Anlage abgeführt werden.

Das im Abscheider 9 erhaltene Flüssigkeitsgemisch, das im wesentlichen aus Dialkylcarbonat, Wasser (H₂O) und Alkanol besteht, wird aus dem Abscheider über den Auslaß 12 entnommen, mittels Druckregelventil 15 entspannt und zur Trennung in seine Einzelkomponenten in der Destillationsanlage 13 destilliert. Die Art des angewandten Destillationsverfahrens ist nicht Gegenstand der vorliegenden Erfindung. Es kann allerdings nach den üblichen Destillationsmethoden des Standes der Technik, beispielsweise Destillation unter erhöhtem Druck oder bei Atmosphärendruck, durchgeführt werden. Vorteilhaft wird das erhaltene Gemisch nach der in US-A 4 162 200 beschriebenen Methode der Extraktionsdestillation aufgetrennt, wobei als Extraktionsmittel vorzugsweise Cyclohexan oder Chlorbenzol verwendet wird.

Das bei der Destillation isolierte Dialkylcarbonat ist im allgemeinen von ausreichender Reinheit, um dem größten Teil seiner Verwendungszwecke direkt zugeführt zu werden. Das zurückgewonnene Alkanol kann gewünschtenfalls wieder vollständig über die Leitung 14 und den Einlaß 3 in den Reaktor zurückgeführt werden. Die Flüssigkeitsmenge im Reaktor wird nach Maßgabe des Alkanolverbrauches und -austrages durch Zuleitung frischen oder rückgeführten Alkanols ergänzt. Zweckmäßigerweise wird die Flüssigkeitsmenge im Reaktor auf diese Weise in etwa konstant gehalten, Schwankungen der Flüssigkeitsmenge sind jedoch möglich und ändern die Ergebnisse nicht signifikant.

Neben der bereits erwähnten, verbesserten Raum-Zeit-Ausbeute hat das erfindungsgemäße Verfahren den besonderen Vorteil, daß der extrem korrosive und nur teilweise im Alkanol gelöste Katalysator während der kontinuierlichen Umsetzung im Reaktor verbleibt. Damit entfallen alle Probleme die beim Arbeiten mit einem speziellen Katalysatorkreislauf überwunden werden müßten, wie Korrosion an den Abtrennvorrichtungen, Abdichtungsschwierigkeiten bei Sonderwerkstoffen, Erosion, Verstopfungen usw. Beim erfindungsgemäßen Verfahren muß daher lediglich der Reaktor mit Sonderwerkstoffen wie Industriekeramik, Email, Teflon oder Glas ausgestattet sein. Dadurch werden die Kosten dieses Verfahrens im Vergleich zu den bereits bekannten Verfahren deutlich gesenkt.

### Beispiel 1

### Herstellung von Dimethylcarbonat

In einer der Zeichnung entsprechenden Anlage wurde Methanol bei 25 bar und 120°C mit Kohlenmonoxid und Sauerstoff umgesetzt. Als Katalysator wurde Kupfermethoxychlorid in einer Menge von 0,7 mol/l Methanol verwendet. Als Reaktor wurde ein Blasenreaktor aus druckstabilem Glas (Volumen 0,13 l) mit einer Rühreinrichtung eingesetzt. Der Reaktor wurde in Sumpffahrweise betrieben und das Reaktionsmedium durch Rühren und durch die Strömung der Reaktionsgase in Bewegung gehalten.

Bei einem CO/O₂-Durchfluß von 60 l CO + 3 l O₂/h und l Reaktionsmischung wurde eine Raum-Zeit-Ausbeute von 25 - 32 g Dimethylcarbonat/(h·l) erzielt. Nach 100 Betriebsstunden wurde bei dem Katalysator noch kein Aktivitätsverlust beobachtet.

Das gebildete Dimethylcarbonat wurde vom Strom der nicht umgesetzten Reaktionsgase zusammen mit dem Reaktionswasser und der zur Erzeugung des ternären Azeotrops notwendigen Menge an Methanol gasförmig aus der Reaktionsmischung ausgetragen. Dieser Gasstrom wurde in einem Abscheider bei 5°C in seine flüssigen und gasförmigen Bestandteile zerlegt. Der flüssige Reaktionsaustrag enthielt Dimethylcarbonat (17 Gew.-%), Wasser (3 bis 5 Gew.-%) und Methanol (78 bis 80 Gew.-%) sowie in Spuren (< 0,2 Gew.-%) Formaldehyddimethylacetal. Das Methanol wurde bei dem Umsatz von 13 % mit einer Selektivität von 98 bis 100 % zu Dimethylcarbonat umgesetzt. Das über den Kopf des Abscheiders abgezogene Kohlenmonoxid/Sauerstoff-Gemisch (60 - 65 l/h) enthielt 0,4 - 3 % O₂ und weniger als 0,05 % CO₂. Der Rest bestand aus CO.

### Beispiel 2

Es wurde analog Beispiel 1 gearbeitet, der CO/O₂-Durchfluß allerdings auf 110 l CO + 2,6 l O₂/h und l Reaktionsmischung eingestellt. Unter diesen Bedingungen wurde die Raum-Zeit-Ausbeute von 54 g Dimethylcarbonat/(h·l) erhalten. Bei einem Methanolumsatz von 16 bis 17 % war die Selektivität für Dimethylcarbonat praktisch quantitativ. Das bei der Kondensation verbleibende Abgas enthielt nur CO (97,6 - 99,6 Vol.-%) und O₂ (0,4 - 2,4 Vol.-%) und konnte wieder zurückgeführt werden. Die Bildung von Nebenprodukten wie Kohlendioxid, Methylchlorid oder Dimethylether wurde nicht beobachtet.

## Patentansprüche

1. Verfahren zur kontinuierlichen Herstellung von Dialkylcarbonaten der allgemeinen Formel I 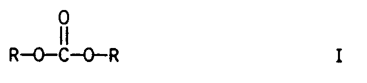 worin R eine C₁- bis C₄-Alkylgruppe ist, durch Umsetzung von C₁- bis C₄-Alkanolen mit Kohlenmonoxid und Sauerstoff in Gegenwart eines im Reaktionsmedium gelösten oder suspendierten, kupferhaltigen Katalysators, bei erhöhter Temperatur und bei erhöhtem Druck, dadurch gekennzeichnet, daß man
a) einen Kohlenmonoxid- und Sauerstoff-enthaltenden Gasstrom in einer Menge von 1 bis 100 Nl/h pro g im Reaktor als Kupferkatalysator vorhandenen Kupfers durch das im Reaktor befindliche Alkanol-Katalysator-Gemisch leitet, wobei ein Teil dieses Gasstromes mit dem Alkanol zu Dialkylcarbonat und Wasser umgesetzt wird und mit Hilfe des restlichen, nicht umgesetzten Kohlenmonoxid/Sauerstoff-Gasstroms das gebildete Dialkylcarbonat und Wasser laufend zusammen mit dem Alkanol, gasförmig aus der Reaktionsmischung austrägt, anschließend
b) das erhaltene, gasförmige Gemisch in einem Abscheider in eine gasförmige und eine flüssige Phase zerlegt, die gasförmige Phase gewünschtenfalls wieder in den Reaktor zurückführt und
c) die im wesentlichen aus Dialkylcarbonat, Wasser und Alkanol bestehende flüssige Phase in ihre Bestandteile trennt, das Dialkylcarbonat isoliert, das Alkanol gewünschtenfalls wieder in den Reaktor zurückführt und
d) die Flüssigkeitsmenge im Reaktor durch Zuleitung frischen oder zurückgeführten Alkanols, nach Maßgabe des Alkanolverbrauches und -austrages ergänzt.

2. Verfahren nach Anspruch 1, dadurch gekennzeichnet, daß man ein Molverhältnis von Alkanol/kupferhaltigem Katalysator von 3/1 bis 330/1 anwendet.

3. Verfahren nach Anspruch 1, dadurch gekennzeichnet, daß man ein CO/O₂-Gemisch verwendet, in dem das Molverhältnis CO/O₂ 1/0,01-0,5 beträgt.

4. Verfahren nach Anspruch 1, dadurch gekennzeichnet, daß man als Katalysator Kupfer(methoxy)chlorid verwendet.

5. Verfahren nach Anspruch 1, dadurch gekennzeichnet, daß man als Katalysator Kupfer(I)chlorid verwendet.

6. Verfahren nach Anspruch 1, dadurch gekennzeichnet, daß man als Alkanol z. B. Methanol verwendet.

## Claims

1. A process for continuously preparing dialkyl carbonates of the general formula I 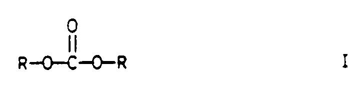 where R is a C₁- to C₄-alkyl group, by reacting C₁- to C₄-alkanols with carbon monoxide and oxygen in the presence of a copper-containing catalyst which is dissolved or suspended in the reaction medium, at elevated temperature and at elevated pressure, which comprises
a) passing a carbon monoxide- and oxygen-containing gas stream through the alkanol-catalyst mixture present in the reactor at a rate of 1 to 100 Nl/h per g of copper present in the reactor as copper catalyst, a part of this gas stream being reacted with the alkanol to give dialkyl carbonate and water, and, with the aid of the remaining, unreacted carbon monoxide/oxygen gas stream, constantly removing from the reaction mixture in gaseous form, together with the alkanol, the dialkyl carbonate and water formed, then
b) dissociating the gaseous mixture obtained into a gaseous and a liquid phase in a separator, if desired recycling the gaseous phase back into the reactor again and
c) separating the liquid phase consisting essentially of dialkyl carbonate, water and alkanol into its constituents, isolating the dialkyl carbonate, if desired recycling the alkanol back into the reactor again and
d) supplementing the amount of liquid in the reactor by passing in fresh or recycled alkanol, according to the consumption and removal of alkanol.

2. A process as claimed in claim 1, wherein a molar ratio of alkanol/copper-containing catalyst of 3:1 to 330:1 is used.

3. A process as claimed in claim 1, wherein a CO/O₂ mixture is used in which the molar ratio of CO/O₂ is 1:0.01-0.5.

4. A process as claimed in claim 1, wherein the catalyst used is copper (methoxy)chloride.

5. A process as claimed in claim 1, wherein the catalyst used is copper (I) chloride.

6. A process as claimed in claim 1, wherein the alkanol used is e.g. methanol.

## Revendications

1. Procédé de préparation continue de carbonates de dialkyle de la formule générale I 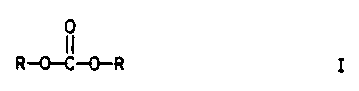 dans laquelle R représente un radical alkyle en C₁ à C₄, par la réaction d'alcanols en C₁ à C₄ avec le monoxyde de carbone et l'oxygène, en présence d'un catalyseur contenant du cuivre, dissous ou mis en suspension dans un milieu de réaction, à température élevée et à pression élevée, caractérisé en ce que
a) on fait passer un courant de gaz contenant du monoxyde de carbone et de l'oxygène au débit de 1 à 100 litres normaux/h par gramme de cuivre présent sous forme de catalyseur au cuivre dans le réacteur, à travers un mélange d'alcanol et de catalyseur se trouvant dans le réacteur, si bien qu'une partie de ce courant de gaz est converti avec l'alcanol en carbonate de dialkyle et en eau et, à l' aide du courant de gaz oxygène/monoxyde de carbone non entré en réaction, on sépare le carbonate de dialkyle formé et l'eau, s'écoulant ensemble avec l'alcanol, sous forme gazeuse, du mélange réactionnel, ensuite
b) on divise le mélange gazeux obtenu dans un séparateur en une phase gazeuse et une phase liquide, on ramène éventuellement la phase gazeuse dans le réacteur et
c) on sépare la phase liquide essentiellement constituée de carbonate de dialkyle, d'eau et d'alcanol en ses constituants, on isole le carbonate de dialkyle, on ramène éventuellement l'alcanol dans le réacteur et
d) on regarnit la quantité de liquide dans le réacteur par l'addition d'alcanol frais ou recyclé, en fonction de la consommation de l'alcanol et de celui qui sort de l'installation.

2. Procédé suivant la revendication 1, caractérisé en ce que l'on utilise un rapport molaire alcanol/catalyseur contenant du cuivre qui varie de 3/1 à 330/1.

3. Procédé suivant la revendication 1, caractérisé en ce que l'on utilise un mélange CO/O₂ dans lequel le rapport molaire CO/O₂ est de 1/0,01-0,5.

4. Procédé suivant la revendication 1, caractérisé en ce que l'on utilise du (méthoxy)chlorure de cuivre à titre de catalyseur.

5. Procédé suivant la revendication 1, caractérisé en ce que l'on utilise du chlorure de cuivre (I) à titre de catalyseur.

6. Procédé suivant la revendication 1, caractérisé en ce que l'on utilise par exemple le méthanol à titre d'alcanol.
